# EUROPEAN PATENT SPECIFICATION

(11) **EP 0 751 756 B1**
(45) Date of publication and mention of the grant of the patent: **29.07.1998**
(21) Application number: 95920840.6
(22) Date of filing: 19.05.1995
(51) Int. Cl.: A61F 13/00

(54) **ABSORBENT SANITARY PRODUCT, IN PARTICULAR AN INCONTINENCE NAPKIN, DIAPER OR THE LIKE**
ABSORBIERENDES HYGIENISCHES PRODUKT, INSBESONDERE EIN INKONTINENZPRODUKT
PRODUIT SANITAIRE ABSORBANT, EN PARTICULIER COUCHE POUR PERSONNE SOUFFRANT D'INCONTINENCE, COUCHE POUR BEBE OU PRODUIT SIMILAIRE

(30) Priority: 21.05.1994 DE 4417946
(43) Date of publication of application: 08.01.1997
(73) Proprietor: THE PROCTER & GAMBLE COMPANY, Cincinnati, Ohio 45202 (US)
(72) Inventor: WILLMS, Eric, D-90443 Nürnberg (DE); SCHÖNEBAUM, Andreas, D-90429 Nürnberg (DE); ZIMMER, Dagmar, D-91161 Hilpoltstein (DE)
(74) Representative: Hübner, Gerd, Dipl.-Phys.
(86) International application number: EP9501912
(87) International publication number: WO9531952

(56) References cited:
- EP-A- 0 590 675
- WO-A-93/10733
- US-A- 4 904 251

## Description

The invention refers to an absorbent sanitary product, in particular an incontinence napkin, diaper or the like with the features mentioned in the preamble of claim 1.

Sanitary products of the generic type are well-known and usually provided with a liquid-impervious underwear-protecting layer for instance produced of a thin polyethylene film contacting the clothes of the user.

Furthermore it is provided with a liquid-permeable, body-contacting covering layer, for instance of hydrophylic nonwoven. An absorbent body is inserted between these two layers, usually consisting of cellulose fluffs comprising super-absorbing particles dispersed therein. The absorbent body can be of varied forms, for instance it is strictly rectangular or - to better suit the anatomic needs - it is of an hourglass form, so that the absorbent body in the gore area has a minimum width and runs straight to the front and to the back end parallel to the longitudinal direction of the sanitary product.

Modern sanitary products are also equipped with two so-called cuffs that run on each longitudinal side of these products, the foot portion of these cuffs being attached to the covering layer and the head portion being provided with elastic straightening elements, for instance by means of elastic rubber threads. These cuffs substantially improve the leakage protection of pertinent sanitary products.

Examples for such sanitary products are given in WO-A-9310733, US-A-4904251 and EP-A-0590675.

Usually - as can be seen in US-A-4904251 - the cuffs are straight in the longitudinal direction of common sanitary products, it being possible to provide cuffs that are relatively close to each other and run on the absorbent body over their entire length, even in the constricted gore area of the sanitary product. Other cuffs are known which are still straight but placed closer to the sides, thus extending, at least in the sanitary product's constricted gore area, along the outside of the absorbent body on the projecting edges of the underwear-protecting layer and cover layer interconnected there (see e.g. EP-A-0590675).

The cuffs first mentioned, which are relatively close to each other, have the disadvantage that a considerable portion of the absorptive surface of the absorbent body lies beside the reach of the cuffs and therefore can not be utilized for the direct absorption of body fluids. This disadvantage also applies to those sanitary products having cuffs situated closer to the sides, since a considerable portion of the surface of the absorbent body is also located to the side of the cuffs.

WO-A-9310733 teaches an absorbent article, in which straight or curved cuffs lie externally of the absorbent pad, the curved cuffs being confined to the central, restricted part of the article.

Another disadvantage of common sanitary products is that the cuffs can bend over in an uncontrollable manner - for instance towards the outside - which causes a considerable loss of the sealing efficiency. The prior art solution coping with this deficiency consists in that the cuffs are bent inwards in the area of their longitudinal ends, in which position they are glued onto the covering layer (so-called tack-downs).

A particular shortcoming of common incontinence napkins with cuffs further resides in that in particular bedridden incontinence patients tend to lie on the cuffs, which may cause pressure marks and in the worst case bedsores.

Proceeding from the above-mentioned prior art problems, it is an object of the invention to improve an absorbent sanitary product of the generic type so that the cuffs can fulfill their sealing function in an optimal manner without impairing the absorptive capacity of the absorbent body and the wearing comfort.

This object is achieved by the features cited in the characterising part of claim 1. The according teaching is a particularly clever profiling of the longitudinal edges of the absorbent body, which in combination with the curved course of the cuffs' foot parallel to said longitudinal edge will lead to special advantages. Tests have revealed that the indicated profiling results in a high wearing comfort and a good absorbing capacity, since an especially large width of the absorbent body is available before and behind the gore area due to the maximum of width according to the invention. This maximum of width also increases the wearing comfort, firstly because the absorbent body's edges are situated far to the side of especially the exposed body parts of bedridden patients, where the pressure of the body resting on the mattress has already considerably decreased. Furthermore, the cuff takes the optimal course since its foot follows the associated longitudinal edge of the absorbent body, so that the cuffs automatically stand upright in the constricted gore area, thus optimally suiting a wearer's gore area. This guarantees a good sealing function.

With the provided course of the absorbent body and the cuff's foot to both the ends, the cuff automatically bends towards the inside and forms a type of pocket, which proves to be optimal concerning the acceptance of body fluids and excrements. This will be explained in detail, taken in conjunction with the exemplary embodiment.

Summing up, it can be said that the absorbent body and the cuffs have a shape which comes up with physical requirements and guarantees a perfect fit and the tightness of the absorbent sanitary product.

Other features, details, advantages and preferred embodiments of the present invention will become apparent from the subclaims and the following description of an example of embodiment, taken in conjunction with the drawings in which
- Fig. 1: is a perspective view of an incontinence napkin according to the invention, and
- Fig. 2: is a diagrammatic cross section according to the line II-II of Fig. 1.

As can be seen in the drawings, the incontinence napkin is provided with a liquid-proof underwear-protecting layer 1 of a thin polyethylene film contacting the clothes of the user. A liquid-permeable covering layer 2 of hydrophilic nonwoven facing the wearer's body is provided. Between said layers 1 and 2 an absorbent body 3 is inserted, which consists of a base pad 4 which faces the underwear-protecting layer 1 and a covering pad 5 attached on top of the base pad 4 and facing the covering layer 2. The base pad 4 consists of common cellulose fluffs, while the covering pad 5, as a so-called "nest of swelling particles", can for instance be of an air-laid nonwoven material, mixed with super-absorbing particles not explicitly shown. Other material combinations are known from the prior art and can also be utilized.

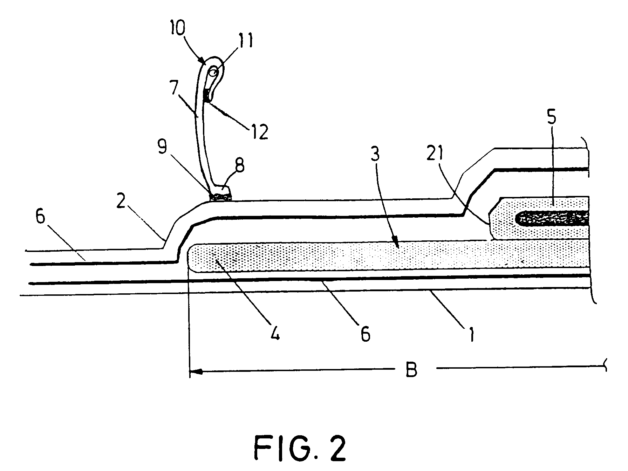

As seen in particular in Fig. 2, the edges of the underwear-protecting layer 1 and of covering layer 2 project from the absorbent body on all sides and are interconnected in the projecting areas by layers of glue 6. These glue layers 6 also extend between the underwear-protecting layer 1 and base pad 4 on the one hand and the covering pad 5 and covering layer 2 on the other hand.

The incontinence napkin's structure and the materials used described so far are completely common and require no further explanation.

On the covering layer 2, cuffs 7, 7' are provided on each of the longitudinal edges, which cuffs 7, 7' consist of a narrow strip of hydrophobic nonwoven fabric. The foot 8 of each cuff 7, 7' is formed as a marginal strip of the cuff bevelled to the inside, which is durably attached to the covering layer 2 by a glue layer 9. The foot 8, bevelled to the inside, supports the rectangularly straightened position of the cuffs 7, 7' shown in Fig. 2.

On the top 10 of the cuffs 7, 7', the marginal strip constituting the latter is bent over and encloses an elastic thread 11, which reaches over the entire length of the cuffs 7, 7' in their longitudinal direction. The bent top 10 of the cuffs 7, 7' is fastened by means of another glue connection 12, which also holds the elastic thread. The elastic thread 11 serves to straighten the cuffs 7, 7' in a way still to be explained in detail.

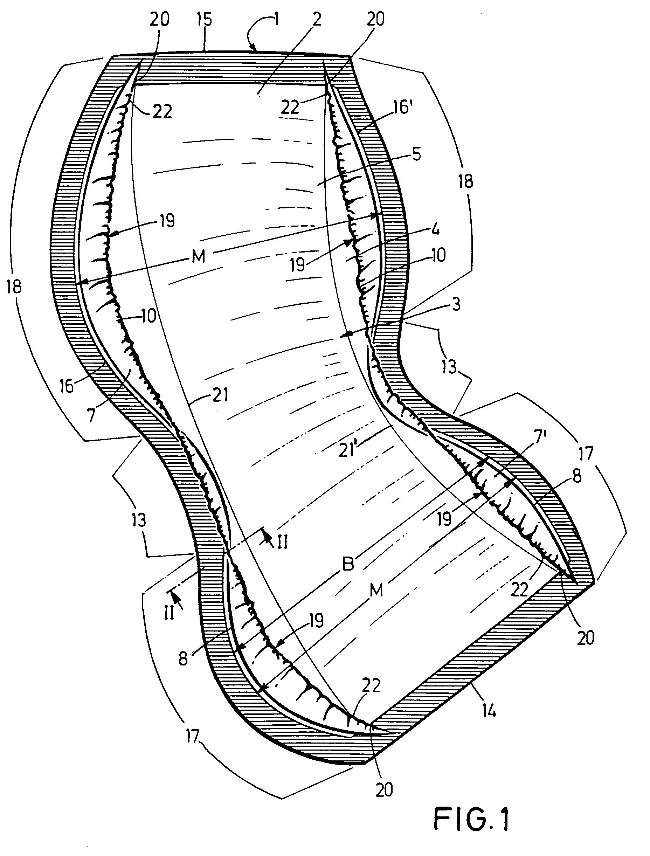

Fig. 1 shows that absorbent body 3 is constricted in the base pad area 4 so that the absorbent body in the gore area 13 has a minimum width B. Between this constricted gore area 13 and the front and back end 14 and 15, the absorbent body 3 now has a maximum M of width B. Directing to the front and back end 14 and 15, the absorbent body 3 gradually decreases in width B. The longitudinal edges 16 and 16' of absorbent body 3 - i.e. of the base pad 4 - therefore describe a wavy line, which beginning from front end 14 forms a convex area 17, the concavely bent gore area 13 and another convex area 18.

As perceptible from Figs. 1 and 2, the foot 8 of each cuff runs parallel to the associated longitudinal edge 16, 16' of the absorbent body over the entire length of the incontinence napkin and has a curved configuration. So the foot 8 follows the course of the curve of the related longitudinal edge 16, 16' of absorbent body 3. The foot 8 of the cuffs 7, 7' is placed in direct vicinity to the associated longitudinal edge 16, 16' of base pad 4 on the latter, which means inside of the longitudinal edges 16, 16'. The optimal maximum space between the foot 8 and the associated longitudinal edge 16, 16' should be 10 mm.

Fig. 1 also shows that the elastic threads 11 in the cuffs 7, 7' have a special effect due to the course of the foot 8 of the cuffs 7, 7'. By means of these elastic threads 11 tensile forces Z are being exercised on to the cuffs 7, 7' with the effect that the top 10 of the cuff 7, 7' is being shortened in the longitudinal direction. This can be noticed by the gathers in the top area. Due to this shortening, the top 10 is located in the convex areas 17, 18 within the foot 8 of the cuffs 7, 7' and in the gore area on the outside. In the maximum area M of width B of the absorbent body 3 - i.e. in front of and behind the gore area 13 the cuffs 7, 7' will neatly bend towards the inside and form a type of pocket 19. To achieve this it is sufficient to glue the longitudinal ends 20 of cuffs 7, 7' only in the foot 8 area to the covering layer 2. The previously mentioned tack-downs can be neglected, which facilitates the production of the object of the invention. Furthermore, the tensile forces Z pull the top ends 22 in the longitudinal direction of the napkin towards the inside, so that the end edges 23 of cuffs 7, 7' run out relatively flat and at an acute angle towards their feet.

In the gore area 13 the tensile forces Z of the elastic threads 11 have the effect that the cuffs 7, 7' automatically take a rectangular upright position, which results in excellent sealing features.

As noticeable in Fig. 1, the cover pad 5 of the absorbent body 3 has a rectangular shape and its straight longitudinal edges 21, 21' clearly lie inside of the longitudinal edges 16, 16' of the base pad 4 and especially of the course line of tops 10 of the cuffs 7, 7'.

## Claims

1. An absorbent sanitary product, in particular incontinence napkin, diaper or the like, comprising
- a liquid-impervious underwear-protecting layer (1), in particular out of film or the like, contacting the clothes of the user,
- a liquid-permeable, body-contacting covering layer (2), in particular of hydrophilic nonwoven material, attached to the edges of the underwear-protecting layer (1)
- an absorbent body (3), constricted in its gore area (13), between underwear-protecting layer (1) and covering layer (2), and
- two cuffs (7, 7') running on each side of the sanitary product in its longitudinal direction, which are attached to the covering layer (2) in their foot area (foot 8) and are provided with elastic straightening elements (11) in the top area (10), wherein
- the absorbent body has a maximum (M) of its width (B) between the constricted gore area (13) on the one hand and the front and back end (14, 14') on the other hand, from where it gradually decreases in width (B) towards its front and back end (14, 15), defining longitudinal edges (16, 16') of the absorbent body (3) to form a wavy line.
characterized in that: the foot (8) of the cuffs (7, 7') takes a curved course over the entire length of the sanitary product parallel to the associated longitudinal edge (16, 16') of the absorbent body (3) and extends at a lateral space of preferably 0 to 10 mm maximum in direct vicinity to the associated longitudinal edge (16, 16') of the absorbent body (3) on the latter.

2. A sanitary product according to claim 1, characterized in that the foot (8) of the associated cuff (7, 7') is structured as a marginal strip of the cuff, bent to the inside of the cuff (7, 7'), and is durably attached, in particular glued, to the covering layer (2).

3. A sanitary product according to claim 1 or 2, characterized in that the longitudinal ends (20) of the cuffs (7, 7') are attached to the covering layer (2) only in the area of the foot (8).

4. A sanitary product according to one of claims 1 to 3, characterized in that an additional absorbent pad (5) is arranged on top of the absorbent body (4), the longitudinal edges (21, 21') of the additional absorbent pad (5) running between the feet (8) of the cuffs (7, 7').

## Patentansprüche

1. Ein absorbierendes Hygieneprodukt, insbesondere eine Inkontinenzvorlage, Windel oder dergleichen, welches umfaßt
- eine flüssigkeitsundurchlässige Unterwäsche-schützende Schichte (1), insbesondere aus Folie oder dergleichen, welche die Kleidung des Verwenders berührt,
- eine flüssigkeitsdurchlässige Körper-berührende Deckschichte (2), insbesondere aus hydrophilem Faservliesmaterial, welche an den Rändern der Unterwäsche-schützenden Schichte (1) fixiert ist,
- einen absorbierenden Körper (3), welcher in seinem Zwickelbereich (13) zusammengezogen ist, zwischen der Unterwäsche-schützenden Schichte (1) und der Deckschichte (2) und
- zwei Bündchen (7, 7'), welche an jeder Seite des Hygieneprodukts in deren Längsrichtung verlaufen, welche an der Deckschichte (2) an ihrer Fußzone (Fuß 8) fixiert sind und mit elastischen geraderichtenden Elementen (11) in der oberen Zone (10) versehen sind, wobei der absorbierende Körper zwischen der eingezogenen Zwickelzone (13) einerseits und dem vorderen und dem hinteren Ende (14, 14') andererseits ein Maximum (M) seiner Breite (B) auf-weist, von wo es graduell in der Breite (B) gegen seine vorderen und hinteren Enden (14, 15) abnimmt, Längsränder (16, 16') des absorbierenden Körpers (3) definierend, um eine wellige Linie zu bilden, dadurch gekennzeichnet, daß der Fuß (8) der Bündchen (7, 7') einen gekrümmten Verlauf über die gesamte Länge des Hygieneprodukts parallel zum zugeordneten Längsrand (16, 16') des absorbierenden Körpers (3) nimmt und sich in einem Querzwischenraum von vorzugsweise 0 bis 10 mm Maximum in direkter Nachbarschaft des zugeordneten Längsrandes (16, 16') des absorbierenden Körpers (3) an Letzterem erstreckt.

2. Ein Hygieneprodukt nach Anspruch 1, dadurch gekennzeichnet, daß der Fuß (8) des zugeordneten Bündchens (7, 7') als ein Randstreifen des Bündchens, gebogen zur Innenseite des Bündchens (7, 7') strukturiert ist und an der Deckschichte (2) dauerhaft fixiert, insbesondere geleimt, ist.

3. Ein Hygieneprodukt nach Anspruch 1 oder 2, dadurch gekennzeichnet, daß die Längsenden (20) der Bündchen (7, 7') an der Deckschichte (2) lediglich in der Zone des Fußes (8) fixiert sind.

4. Ein Hygieneprodukt nach einem der Ansprüche 1 bis 3, dadurch gekennzeichnet, daß ein zusätzliches absorbierendes Kissen (5) obenauf am absorbierenden Körper (4) angeordnet ist, wobei die Längsränder (21, 21') des zusätzlichen absorbierenden Kissens (5) zwischen dem Fuß (8) der Bündchen (7, 7') verlaufen.

## Revendications

1. Produit hygiénique absorbant, en particulier serviette pour incontinents, couche-culotte, ou analogue, comprenant :
- une couche de protection de sous-vêtement (1) imperméable aux liquides, en particulier réalisée en un film ou analogue, en contact avec les vêtements de l'utilisateur,
- une couche de recouvrement en contact avec le corps (2) perméable aux liquides, en particulier réalisée en un matériau non tissé hydrophile, fixée aux bords de la couche de protection de sous-vêtement (1),
- un corps absorbant (3), resserré dans sa zone du gousset (13), entre la couche de protection de sous-vêtement (1) et la couche de recouvrement (2), et
- deux revers (7, 7') s'étendant sur chaque côté du produit hygiénique dans sa direction longitudinale, qui sont fixés à la couche de recouvrement (2) dans leur zone de pied (pied 8) et sont pourvus d'éléments de redressement élastiques (11) dans la zone supérieure (10), dans lequel :
le corps absorbant présente un maximum (M) de sa largeur (B) entre la zone du gousset resserrée (13) d'une part et l'extrémité avant et arrière (14, 14') d'autre part, à partir duquel sa largeur (B) diminue progressivement vers son extrémité avant et arrière (14, 15) définissant des bords longitudinaux (16, 16') du corps absorbant (3) pour former une ligne ondulée,
caractérisé en ce que
le pied (8) des revers (7, 7') prend un tracé incurvé sur l'entière longueur du produit hygiénique parallèle au bord longitudinal associé (16, 16') du corps absorbant (3) et s'étend à une distance latérale, de préférence de 0 à 10 mm maximum, directement au voisinage du bord longitudinal associé (16, 16') du corps absorbant (3) sur ce dernier.

2. Produit hygiénique selon la revendication 1, caractérisé en ce que le pied (8) du revers associé (7, 7') est structuré comme une bande marginale du revers, cintrée vers l'intérieur du revers (7, 7'), et est fixé de façon durable, en particulier collé, à la couche de recouvrement (2).

3. Produit hygiénique selon la revendication 1 ou la revendication 2, caractérisé en ce que les extrémités longitudinales (20) des revers (7, 7') ne sont fixées à la couche de recouvrement (2) que dans la zone du pied (8).

4. Produit hygiénique selon une des revendications 1 à 3, caractérisé en ce qu'un tampon absorbant supplémentaire (5) est placé sur la partie supérieure du corps absorbant (4), les bords longitudinaux (21, 21') du tampon absorbant supplémentaire (5) s'étendant entre les pieds (8) des revers (7, 7').
